# EUROPEAN PATENT APPLICATION

(11) **EP 4 797 276 A1**
(43) Date of publication of application: **26.08.2026**
(21) Application number: 25305227.8
(22) Date of filing: 19.02.2025
(51) Int. Cl.: G16H 30/40, G16H 50/30, G16H 50/70, G01N 33/50, G01N 33/574

(54) **DEVICE AND METHOD FOR DETERMINING A VALUE QUANTIFYING A RISK OF RELAPSE OF BREAST CANCER FOR A PATIENT**

(71) Applicant: Spotlight Medical, 75014 Paris (FR)
(72) Inventor: LEROUSSEAU, Marvin, 75014 Paris (FR); FRECHIN, Lucie, 75014 Paris (FR)
(74) Representative: Plasseraud IP

(57) **Abstract**

A device for determining a value quantifying a risk of relapse of breast cancer for a patient comprises a data storage adapted to store patient data associating, for each given patient at a least one whole histological slide image and patient clinical data comprising at least a tumor size, a number of positive lymph nodes, a tumor grade, and progesterone receptor (PR) positivity value, a feature extractor arranged to receive a whole histological slide image and associated patient clinical data, to derive tumor architecture features comprising a tumor area, an invasive tumor nest density, a tumor density at the invasive front, and an in situ tumor nest density, tumor microenvironment features comprising a border composition of in situ tumor, a variance of healthy gland size, an inflammatory stroma area, and an average nuclei size of stromal cells, and mitosis features comprising a mitotic hotspot count and a mitotic density, and to return a patient feature vector comprising said tumor architecture features, said tumor microenvironment features, said mitosis features, and features derived from said associated patient clinical data, a risk classifier using a machine learning module which is arranged to receive a patient feature vector as an input and to return a value quantifying a risk of relapse of breast cancer for a patient, said machine learning module having been trained with a dataset of labelled patient feature vectors and being arranged to return a risk value.

This device is arranged to receive a set of patient data of a given patient, to provide at least some of said given patient's whole histological slide image and said given patient's clinical data as inputs to the feature extractor, to provide at least some of the resulting patient feature vectors to the risk classifier, and to return a value quantifying a risk of relapse of breast cancer for a patient for said given patient based on the outputs of the risk classifier.

## Description

### Technical Field

The present invention relates to cancer medical technology, in particular the invention relates to a device and associated method for training a machine learning model of a risk classifier configured to determine a value quantifying a risk of relapse of a patient afflicted with breast cancer. Furthermore, the invention relates to a device and associated method for determining a value quantifying a risk of relapse of the patient afflicted with breast cancer using the trained machine learning model, and a device for adapting a cancer treatment based on the value quantifying a risk of relapse of the patient afflicted with breast cancer.

### Background

In the past decades, cancer treatments have made tremendous progress. As a result, more and more patients are cured, such that managing relapses has now become a significant issue. In this context, breast cancer is of particular importance, as it is the most common type of cancer. Statistics show that approximately 70% of breast cancers are hormone-sensitive, and that about 20% of these cancers induce metastatic relapse.

### Summary

Detecting which patients are likely to suffer a relapse is a daunting task, especially in view of the risks associated with wrongly predicting a non-relapse. This has led to aggressive treatment strategies for the patients after their initial treatment, which have extremely heavy costs, from a financial perspective as they are very expensive, but more importantly from a health perspective, as they take a serious toll on the patients.

This means that a significant amount of patients who have been cured of their breast cancer end up taking expensive and health degrading treatments that they do not ultimately need, due to the impossibility to reliable predict their chance of relapse.

The invention aims at improving the situation. To this end, the Applicant proposes a device for determining a value quantifying a risk of relapse of breast cancer for a patient comprising:
- a data storage adapted to store patient data associating, for each given patient at a least one whole histological slide image and patient clinical data comprising at least a tumor size, a number of positive lymph nodes, a tumor grade, and progesterone receptor (PR) positivity value,
- a feature extractor arranged to receive a whole histological slide image and associated patient clinical data, to derive tumor architecture features comprising a tumor area, an invasive tumor nest density, a tumor density at the invasive front, and an in situ tumor nest density, tumor microenvironment featuFIGres comprising a border composition of in situ tumor, a variance of healthy gland size, an inflammatory stroma area, and an average nuclei size of stromal cells, and mitosis features comprising a mitotic hotspot count and a mitotic density, and to return a patient feature vector comprising said tumor architecture features, said tumor microenvironment features, said mitosis features, and features derived from said associated patient clinical data,
- a risk classifier using a machine learning module which is arranged to receive a patient feature vector as an input and to return a value quantifying a risk of relapse of breast cancer for a patient, said machine learning module having been trained with a dataset of labelled patient feature vectors and being arranged to return a risk value,
said device being arranged to receive a set of patient data of a given patient, to provide at least some of said given patient's whole histological slide image and said given patient's clinical data as inputs to the feature extractor, to provide at least some of the resulting patient feature vectors to the risk classifier, and to return a value quantifying a risk of relapse of breast cancer for a patient for said given patient based on the outputs of the risk classifier.

This method is advantageous because it allows it performs better than all known methods of predicting breast cancer relapse. Furthermore, it is explainable and interpretable, while relying on data which are routine clinicopathological variables.

In various embodiments, the method may present one or more of the following features:
- said feature extractor comprises a first annotation model comprising a deep learning neural network arranged to receive a whole histological slide image as an input image and to return a class identifier for each pixel of the input image, said class identifier being chosen in a group comprising non-tissue, invasive tumor, in situ tumor, healthy epithelium, necrosis, inflamed stroma, tumor-associated stroma, adipocyte tissue and unclassified tissue, a second annotation model comprising a deep learning neural network arranged to receive a whole histological slide image as an input image and to return data identifying cell nuclei in the input image in the form of a nuclei identifier attached to each pixel of the input image and/or a set of nuclei bounding boxes, and a third annotation model comprising a third deep learning neural network arranged to receive a whole histological slide image as an input image and to return data identifying cell mitosis in the input image in the form of a nuclei identifier attached to each pixel of the input image and/or a set of mitosis bounding boxes, and a feature calculator arranged to derive said tumor architecture features, said tumor microenvironment features, said mitosis features from the outputs of said first annotation model, said second annotation model and said third annotation model provided with the whole histological slide image received by the feature extractor,
- the first annotation model comprises an attention U-Net model with a MobileNet_v3 encoder,
- the second annotation model comprises a NuLite model,
- the third annotation model comprises a Fully Convolutional One-Stage (FCOS) Object Detection model with a ResNet-50 encoder,
- said patient clinical data further comprises one or more of a Ki-67 proliferation index, an estrogen receptor (ER) status, an estrogen receptor (ER) positivity value, a progesterone receptor (PR) status, a HER2 intensity (HER2-) value, a pathological T stage, pathological N stage , a menopausal status, a type of surgery, and a histological subtype,
- when the patient data comprises more than one whole histological slide image, the device is arranged to derive a patient risk value from each risk value derived from the more than one whole histological slide images and to determine a value quantifying a risk of relapse of breast cancer for a patient by comparing said patient risk value to a threshold value, and, when the patient data comprises one whole histological slide image to determine a value quantifying a risk of relapse of breast cancer for a patient by comparing said risk value derived from said one whole histological slide image to a threshold value,
- when the patient data comprises more than one whole histological slide image, the device is arranged to determine said patient risk value by performing one or more of the following operations : discarding certain risk values based on their corresponding patient feature vector, discarding certain risk values based on their value, averaging certain risk values, weighed averaging certain risk values, and
- the machine learning module of the risk classifier comprises a Cox model or a gradient boosted tree type model or a Support Vector Machine or a random forest model.

The invention also concerns for training a machine learning module for use in the above device, comprising:
- a data storage for receiving a training dataset comprising patient feature vectors each labelled with a corresponding risk value,
- a machine learning module comprising a Cox model or a gradient boosted tree type model or a Support Vector Machine or a random forest model arranged to receive a patient feature vector and to return a risk value,
said device being arranged to train said machine learning module with said training dataset.

The invention also concerns a method for determining a value quantifying a risk of relapse of breast cancer for a patient comprising:
a) receiving a set of patient data of a given patient, said patient data comprising at a least one whole histological slide image and patient clinical data comprising at least a tumor size, a number of positive lymph nodes, a tumor grade, and progesterone receptor (PR) positivity value,
b) for at least some of said at least one whole histological slide image, deriving tumor architecture features comprising a tumor area, an invasive tumor nest density, a tumor density at the invasive front, and an in situ tumor nest density, tumor microenvironment features comprising a border composition of in situ tumor, a variance of healthy gland size, an inflammatory stroma area, and an average nuclei size of stromal cells, and mitosis features comprising a mitotic hotspot count and a mitotic density, and returning each time a patient feature vector comprising said tumor architecture features, said tumor microenvironment features, said mitosis features, and features derived from said patient clinical data,
c) providing said patient feature vectors to a machine learning module having been trained with a dataset of labelled patient feature vectors and being arranged to return a risk value quantifying a risk of relapse of breast cancer for a patient, and returning a value quantifying a risk of relapse of breast cancer for a patient for said given patient based on the risk values determined from said patient feature vectors.

In various embodiments, the method may present one or more of the following features:
- operation b) comprises, for said at least some of said at least one whole histological slide image:
   b1) determining a class identifier for each pixel of the input image, said class identifier being chosen in a group comprising non-tissue, invasive tumor, in situ tumor, healthy epithelium, necrosis, inflamed stroma, tumor-associated stroma, adipocyte tissue and unclassified tissue, data identifying cell nuclei in the input image in the form of a nuclei identifier attached to each pixel of the input image and/or a set of nuclei bounding boxes, and data identifying cell mitosis in the input image in the form of a nuclei identifier attached to each pixel of the input image and/or a set of mitosis bounding boxes, and
   b2) deriving said tumor architecture features, said tumor microenvironment features, said mitosis features from the output said at least some of said at least one whole histological slide image and the output of operation b1),
- operation c) comprises, when the patient data comprises more than one whole histological slide image, deriving a patient risk value from each risk value determined from said patient feature vectors and determining a value quantifying a risk of relapse of breast cancer for a patient by comparing said patient risk value to a threshold value, and, when the patient data comprises one whole histological slide image, determining a value quantifying a risk of relapse of breast cancer for a patient by comparing said risk value derived from said one whole histological slide image to a threshold value, and
- operation c) comprises, when the patient data comprises more than one whole histological slide image, determining said patient risk value by performing one or more of the following operations : discarding certain risk values based on their corresponding patient feature vector, discarding certain risk values based on their value, averaging certain risk values, weighed averaging certain risk values.

The invention also concerns non-transitory program storage device comprising instructions which, when executed by a computer, cause the computer to carry out the above method.

Other features and advantages of the invention will readily appear in the following description of the drawings, which show exemplary embodiments of the invention and on which:
- Figure 1 shows a general diagram of a computer device according to an embodiment,
- Figure 2 shows a generic view of a computer program used in the embodiment of figure 1,
- Figure 3 shows an exemplary process executed by the device of the invention,
- Figure 4 shows an exemplary embodiment of the process of figure 3, and
- Figure 5 shows an exemplary whole histological slide image.

The drawings and the following description are comprised for the most part of positive and well-defined features. As a result, they are not only useful in understanding the invention, but they can also be used to contribute to its definition, should the need arise.

The description may make reference or use elements protected or protectable by copyright. The Applicant does not object to the reproduction of those elements in as much as it is limited to the necessary legal publications, however this should not be construed as a waiver of rights or any form of license.

### Detailed description

As explained in the introduction, the invention is directed to patients who have suffered from breast cancer and for whom it is advantageous to try and determine a risk of relapse.

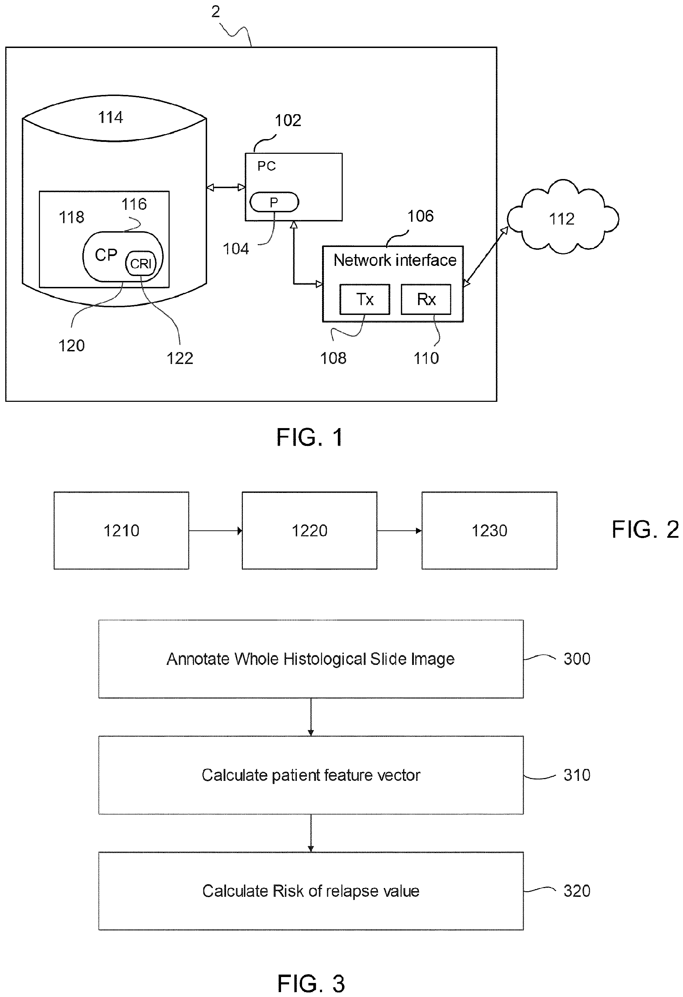

Figure 1 shows a general diagram on an embodiment of the computer device according to the invention.

The computer device shown on Figure 1 as a block diagram of a device 2 (*e.g.,* a network node, connected device, and the like), according to an embodiment. As shown in figure 1 the device may comprise: processing circuitry (PC) 102, which may include one or more processors (P) 104; a network interface 106 comprising a transmitter (Tx) 108 and a receiver (Rx) 110 for enabling the device to transmit data to and receive data from other computing devices connected to a network 112 (e.g., an Internet Protocol (IP) network) to which network interface 106 is connected; and a local storage unit (a.k.a., "data storage system") 114, which may include one or more non-volatile storage devices and/or one or more volatile storage devices.

In embodiments where PC 102 includes a programmable processor, a computer program product (CPP) 116 may be provided. CPP 116 includes a computer readable medium (CRM) 118 storing a computer program (CP) 120 comprising computer readable instructions (CRI) 122. CRM 118 may be a non-transitory computer readable medium, such as, magnetic media (e.g., a hard disk), optical media, memory devices (e.g., random access memory, flash memory), and the like.

Processors 104 include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In an embodiment, data storage 114 stores patient data. The patient data may be comprised of data of two natures: one or more whole histological slide images, and patient clinical data. The data storage 114 may also store patient feature vectors which are derived from the patient data.

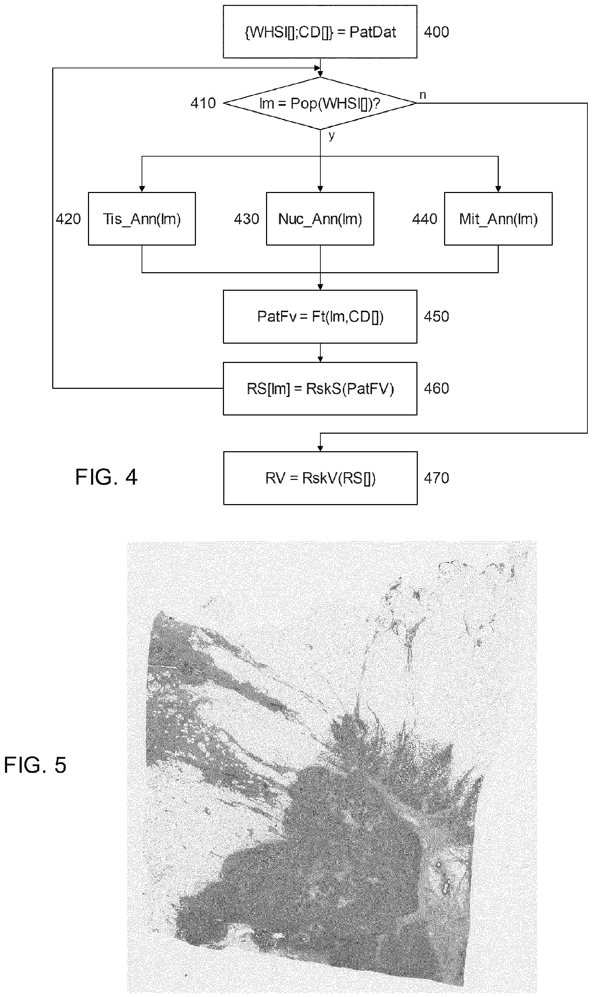

Figure 5 shows an example of a whole histological slide image. In some embodiments, patient clinical data comprises a tumor size, a number of positive lymph nodes, a tumor grade, and progesterone receptor (PR) positivity value (i.e. a percentage of tumor cells positive to progesterone receptor). In other embodiments, patient clinical data may further comprise one or more of a Ki-67 proliferation index, an estrogen receptor (ER) status, an estrogen receptor (ER) positivity value, a progesterone receptor (PR) status, a HER2 intensity (HER2-) value, a pathological T stage, a pathological N stage, a menopausal status, a type of surgery, and a histological subtype.

As will appear below, the patient feature vectors derive from the patient data, and more specifically from specifically chosen features which are obtained from the whole histological slide images and from the patient clinical data. In some embodiments, the features obtained from the whole histological slide images are obtained by deep learning based annotations of these images, and by processing of the annotated images. Such annotated images may be stored in data storage 114.

The Applicant has discovered that some features are particularly critical in the goal of predicting a risk of relapse of a breast cancer patient. This has allowed to develop a model using a limited number of data and which allows to reliably predict a non-relapse, and more specifically data which is obtained routinely in patient follow-up. This means that the Applicant's model can be widely used without any modification to the collection of clinicopathological data, while offering guarantee that a patient will not undergo a relapse and that their post treatment can be adapted accordingly.

The features comprised in the patient feature vector belong to four main categories: tumor architecture features, tumor microenvironment features, mitosis features, and patient clinical data associated to the whole histological slide image(s) from which the former derive.

As will appear in more detail below:
- tumor architecture features comprise a tumor area, an invasive tumor nest density, a tumor density at the invasive front, and an in situ tumor nest density,
- tumor microenvironment features comprise a border composition of in situ tumor, a variance of healthy gland size, an inflammatory stroma area, and an average nuclei size of stromal cells, and
- mitosis features comprise a mitotic hotspot count and a mitotic density,
- clinical features are derived from the patient clinical data.

Most of these features have a clearly understood definition in the art. Some ways to derive them will be described below, but it is understood that there may be some alternative manner to derive them without changing the fundamental nature of the data. In other words, the nature of these features is more important than the way in which they are derived from the patient data.

In the example described herein, the data storage 114 may be realized in any way suitable, that is by means of a hard disk drive, a solid-state drive, a flash memory, a disk (CD or DVD), a memory embedded in a processor, a distant storage accessible in the cloud, etc. Data storage 114 may also store any transitory data which may be generated in the course of executing the invention, as well as maps resulting from the operation of the device 2, possibly combined with practitioner made annotations.

Figure 2 shows an exemplary schematic of computer program 120.

The computer program (CP) 120 comprises two main modules which work hand-in-hand:
- An image annotation module 1210,
- A feature vector calculation module 1220, and,
- A risk calculation module 1230.

The image annotation module 1210 and the feature vector calculation module 1220 form together a feature extractor.

The feature extractor is arranged to process patient data in order to obtain a patient feature vector which will contain the features described above. This patient feature vector is extremely advantageous because it constitutes a compressed form of the patient data which is sufficient to qualify a risk of relapse. This qualification is performed by the risk calculation module 1230 which can be whole or part of a risk classifier.

Figure 3 will now be used to describe an exemplary process performed with device 2.

In a first operation 300, patient data is received or accessed in the data storage 114 as input patient data, and its at least one or more whole histological slide image is annotated. This annotation is performed by the image annotation module 1210. The goal of this annotation is to determine data in the at least one or more whole histological slide image which will allow to calculate the tumor architecture features, tumor microenvironment features and mitosis features. In some embodiments, the features could be determined otherwise and/or obtained as inputs.

In the example described herein, the image annotation module 1210 comprises three annotation models which may be run in series or in parallel.

Here, the first annotation model is an attention U-Net model, for example of the type described in the article by Oktay et al., "Attention u-net: Learning where to look for the pancreas", arXiv preprint arXiv:1804.03999, 2018 Apr 11, with a MobileNet V3 encoder by Howard et al., "Searching for mobilenetv3", In Proceedings of the IEEE/CVF international conference on computer vision 2019 (pp. 1314-1324). The first annotation model is arranged to classify all of the pixels in each whole histological slide image into one of nine categories: non-tissue, invasive tumor, in situ tumor, healthy epithelium, necrosis, inflamed stroma, tumor-associated stroma, adipocyte tissue and unclassified tissue. In other embodiments, another deep learning module such as convolutional neural network such as DeepLabV3 or SegNet may be used instead or a transformer such as Vision Transformer (ViT) or Swin Transformer.

Here, the second annotation model is NuLite model (disclosed by Tommasino et al. in "NuLite-Lightweight and Fast Model for Nuclei Instance Segmentation and Classification", arXiv preprint arXiv:2408.01797, 2024 Aug 3) . The second annotation neural network detects every nuclei in the whole histological slide image by assigning a bounding box around each nuclei. Alternatively, the bounding box can be replaced by labelling each pixel with a flag indicating if the pixel belongs to a nuclei or not, with a nuclei identifier for differentiation purposes. In other embodiments, another deep learning module such as convolutional neural network (e.g. Mask R-CNN, Hybrid Task Cascade) may be used instead or a transformer (Mask2Former, Swin Transformer).

Here, the third annotation model is a Fully Convolutional One-Stage (FCOS) Object Detection model (disclosed by Tian et al. in "FCOS: A simple and strong anchor-free object detector", IEEE transactions on pattern analysis and machine intelligence, 2020 Oct 19;44(4):1922-33 with a ResNet-50 encoder (disclosed by He et al. in "Deep residual learning for image recognition", In Proceedings of the IEEE conference on computer vision and pattern recognition 2016 (pp. 770-778)). The third annotation neural network is arranged to detect every mitosis (cell division event) within the tissue, and to correspondingly annotate or label the associated pixels. In other embodiments, another deep learning module such as convolutional neural network (e.g. R-CNN, Fast R-CNN, YOLO, RetinaNet, CornerNet) may be used instead or a transformer (e.g. DETR (DEtection TRansformer), Swin Transformer, DINO).

### Model Training

In the example provide herein, the first annotation model, second annotation model and third annotation model have been trained as described below.

The training of the first annotation model began with the preparation of a dataset that included patches of hematoxylin and eosin (H&E) stained tissue slides at magnification 20x. In this context, a patch refers to a smaller, focused region of a whole slide image (WSI). Each patch had a minimum width of 512 pixels. These patches were manually annotated by 2 pathologists, who labeled each pixel according to the predefined tissue categories (e.g., invasive tumor, necrosis, etc.).

The details of the training dataset, including the number of annotated pixels per class, the number of whole slide images (WSIs) from which these patches were extracted, the number and type of pathologist annotators, and the centers and countries from which the data originated are as follows:
* Total number of training patches = 3111
* Number and source of WSIs from which the training patches were extracted

| Number of WSIs | Number of patients | Number of centers | Location of centers | Scanner used |
|---|---|---|---|---|
| 1024 | 1024 | 31 centers (TCGA) | USA & Europe | Aperio and ScanScope^{®} XT and Leica Aperio AT2 |
| 96 | 64 | 1 (Sunnybrook Health Sciences Centre) | Canada | Aperio and ScanScope^{®} XT |
| 449 | 238 | 1 (National Cancer Institute's cancer Human Biobank (caHUB)) | USA | Leica Aperio AT2 |
| 245 | 245 | 1 (Gustave Roussy) | France | Olympus VS200 |
| 547 | 189 | 1 (Institute for High Performance Computing and Networking) | Italy | Leica Aperio AT2 |
| 93 | 63 | 2 (Radboud University Medical Center & Julius Center at UMC Utrecht) | Netherlands | Hamamatsu NanoZoomer XR and NanoZoomer S360 |

* Number of annotated pixels per class:

| Tissue Class | Total Number of Annotated Pixels | Number of Patches | Number of WSIs |
|---|---|---|---|
| Background (non-tissue) | 1695802193 | 3110 | 2808 |
| Invasive tumor | 374475298 | 1179 | 1179 |
| In situ tumor | 80087976 | 299 | 299 |
| Healthy epithelium (including glands) | 24946086 | 353 | 312 |
| Necrosis | 92819766 | 352 | 296 |
| Inflamed stroma | 48032076 | 449 | 449 |
| Tumor-associated stroma | 151141241 | 887 | 887 |
| Adipocyte tissue | 273039733 | 1767 | 1761 |

The first annotation model was then trained in a fully supervised manner, meaning that it was explicitly taught to predict these annotations by learning from the provided pairs of input patches and their corresponding labeled masks. During training, the model iteratively adjusted its parameters to minimize the difference between its predictions and the expert annotations. The goal of this process was to generalize the model's ability to correctly classify tissue types in new, unseen patches.

The model was optimized using the Adam Weighted Optimizer (Loshchilov et al. in "Decoupled weight decay regularization", arXiv preprint arXiv:1711.05101, 2017, 2017). The optimizer adjusts the learning rate dynamically based on the history of gradients, improving convergence in complex training scenarios such as training neural networks. The model was trained for up to 2000 epochs, with early stopping applied, based on performance measured from a validation subset consisting of 20% of the training patches. This ensured the model stopped training once no significant improvements were observed, preventing overfitting and reducing unnecessary training time.

To better capture the pixel-level differences between predicted and true segmentation masks, the Dice loss function (disclosed by Sudre et al. in "Generalised dice overlap as a deep learning loss function for highly unbalanced segmentations", In Deep Learning in Medical Image Analysis and Multimodal Learning for Clinical Decision Support: Third International Workshop, DLMIA 2017, and 7th International Workshop, ML-CDS 2017, Held in Conjunction with MICCAI 2017, Québec City, QC, Canada, September 14, Proceedings 3 2017 (pp. 240-248). Springer International Publishing) was employed during training. Dice loss is particularly well-suited for tasks like tissue segmentation because it directly measures the overlap between the predicted and true annotations, ensuring better accuracy in identifying tissue boundaries.

To handle the high variability in tissue appearances across different patients, pathological protocols, and staining conditions, on-the-fly data augmentation was applied during training. Data augmentation refers to the process of artificially increasing the diversity of the training dataset by applying random transformations to the patches, enabling the model to learn more generalized features and improve its robustness to unseen data. Tissue appearance can vary due to differences in staining procedures, section thickness, or imaging quality, and data augmentation helps address these variations by exposing the model to a broader set of examples during training. At every training iteration, the parameters of the augmentations were randomly sampled, meaning each patch was randomly transformed. Examples of these parameters include the probability of applying a horizontal flip (e.g., 50%), the degree of rotation (e.g., sampled between 0° and 90°), and the range for brightness adjustment (e.g., ±20%). Example of transformations include horizontal and vertical flips, rotation, shifts (translations), Gaussian blur, contrast, saturation, and brightness adjustments and stain augmentation.

The second annotation model was trained using a similar approach as the tissue segmentation model, with a focus on detecting and segmenting individual nuclei within the annotated tissue regions. As in the first annotation model, training involved extracting patches from whole slide images (WSIs) of hematoxylin and eosin (H&E) stained slides. These patches were manually annotated by 3 pathologists, who provided bounding boxes and pixel-level segmentations for each nucleus.

The second annotation model was trained in a fully supervised manner, where it learned to predict the location and segmentation of nuclei by comparing its predictions to the annotations provided by pathologists. This involved iteratively adjusting the model's parameters to minimize the difference between predicted and annotated segmentations.

The details of the training dataset, including the number of annotated pixels per class, the number of whole slide images (WSIs) from which these patches were extracted, the number and type of pathologist annotators, and the centers and countries from which the data originated are as follows:
* Number of patches = 3111
* Number of annotated nuclei = 270619

| Number of WSIs | Numbe r of patients | Number of centers | Location of centers | Scanner used |
|---|---|---|---|---|
| 1024 | 1024 | 31 centers (TCGA) | USA & Europe | Aperio ScanScope^{®} XT and Leica Aperio AT2 |
| 41 | 41 | 1 (University Hospitals Coventry and Warwickshire) | UK | Omnyx VL120 scanner |
| 59 | 59 | 1 (Kangbuk Samsung Hospital) | Republic of Korea | Leica Aperio AT2 |

The second annotation model was trained using the same principles applied in the tissue segmentation model, with appropriate changes for instance segmentation tasks. The model's parameters were updated with the Adam Weighted Optimizer (referenced above) and an early stopping strategy was employed. As instance segmentation requires the model to not only locate nuclei but also segment them precisely, the loss function employed was the one defined in Tommasino et al., 2024 referenced above.

The same on-the-fly data augmentation techniques used for the first annotation model were applied to train the second annotation model. These transformations were used to account for variability in nuclei appearances due to differences in tissue staining, slide preparation, and imaging conditions. The same type of transformations were used.

By applying these data augmentation techniques, the second annotation model was trained to handle the wide variability in nuclei appearances across different tissue types and imaging conditions, ensuring that it could robustly detect and segment nuclei in real-world clinical scenarios.

The third annotation model was designed to specifically identify mitotic figures (that is cells undergoing division) within the whole histological slide images. The training process for this model followed a similar approach to the first annotation model and second annotation model. Patches were extracted from whole histological slide images (WSIs) of hematoxylin and eosin (H&E) stained tissue, and had at least 1024 pixels of width. 2 pathologists annotated these patches by marking the location of each mitosis, providing bounding boxes around the cells undergoing division.

The third annotation model was trained in a fully supervised manner, where it learned to detect mitotic figures by comparing its predictions against these pathologist annotations. Through iterative training, the model adjusted its internal parameters to minimize discrepancies between predicted and true mitosis locations.

Here are the details of the training dataset, including the number of annotated pixels per class, the number of whole slide images (WSIs) from which these patches were extracted, the number and type of pathologist annotators, and the centers and countries from which the data originated are as follows:
* Number of patches = 990
* Number of annotated mitoses = 17649
* Number and source of WSIs from which the training patches were extracted:

| Number of WSIs | Number of patients | Number of centers | Location of centers | Scanner used |
|---|---|---|---|---|
| 246 | 246 | 31 centers (TCGA) | USA & Europe | Aperio ScanScope^{®} XT and Leica Aperio AT2 |
| 403 | 403 | 3 (UMC Utrecht & VMU Vienna & FU Berlin) | Netherlands, Austria, and Germany | Hamamatsu XR NanoZoomer 2.0, Aperio ScanScope CS2, and 3DHistech Pannoramic Scan II |
| 449 | 238 | 1 (National Cancer Institute's cancer Human Biobank (caHUB)) | USA | Leica Aperio AT2 |

The mitosis detection model was trained using the Adam Weighted Optimizer (referenced above) and early stopping criteria, similar to the other models in the pipeline. The early stopping strategy was based on a validation subset, ensuring that the model halted training once no further improvements were detected.

For this model, an object detection focal loss function was employed as defined in Tian et al., 2020 referenced above.

The same on-the-fly data augmentation techniques used for the tissue segmentation model and the nuclei instance segmentation model were applied to train the mitosis detection model. These transformations were used to account for variability in mitosis appearances due to differences in tissue staining, slide preparation, and imaging conditions.

Following operation 300, the feature vector calculation module 1220 is called in an operation 310, which may use the annotated images resulting from operation 300 as well as the patient clinical data to generate the patient feature vector.

In this case, the features used by the risk calculation module 1230 are not learned by any machine learning algorithm, but instead are derived from the annotations models. This process involves computing quantitative values based on the results of operation 300. In other words, the resulting features are designed, not learned, and provide a structured way of summarizing the morphological and spatial characteristics of the tissue.

### Feature computation

In the example described herein, the feature vector calculation module 1220 may compute the tumor architecture features, tumor microenvironment features and mitosis features as follows.

The tumor area feature represents the ratio of the area of the viable tumor to the area of the whole tissue. This feature may be computed by dividing the number of pixels within the viable tumor by the total number of pixels within the whole tissue.

The invasive tumor nest density feature represents the number of continuous regions ('nests') of 'invasive tumor' divided by the area of the viable tumor. To compute this feature, first, a binary mask is created where pixels segmented as 'invasive tumor' are set to True and all other pixels are set to False. The binary mask is then labeled to identify connected regions (nests), where each labeled region corresponds to a continuous region of 'invasive tumor' pixels. The number of nests is determined by counting the unique labeled regions and is then divided by the area of the viable tumor (sum of pixels in the viable tumor region) to compute the feature.

The tumor density at the invasive front feature represents the 25th percentile of the distribution of distances from invasive tumor pixels to the border of the viable tumor, normalized by the 25th percentile of distances of all viable tumor pixels to the same border. This feature may be computed by extracting the pixels forming the border of the viable tumor. The distances of all invasive tumor pixels to the border of the viable tumor are then computed, and the 25th percentile of these distances is retrieved. Additionally, the distances of all pixels within the viable tumor to the border are computed, and the 25th percentile of this distribution is extracted for normalization. The final feature is determined by dividing the 25th percentile of the invasive tumor distance distribution by the 25th percentile of the viable tumor distance distribution.

The in situ tumor nest density feature represents the number of continuous regions ('nests') of 'in situ tumor' in the viable tumor divided by the area of the viable tumor. This feature may be computed by first creating a binary mask where pixels segmented as 'in situ tumor' are set to True and all other pixels are set to False. The binary mask is then labeled to identify connected regions (nests), where each labeled region corresponds to a continuous region of 'in situ tumor' pixels. The number of nests is determined by counting the unique labeled regions and is then divided by the area of the viable tumor (sum of pixels in the viable tumor region) to compute the feature.

The border composition of in situ tumor feature represents the percentage of the border of 'in situ tumor' that is occupied by 'tumor-associated stroma'. This feature may be computed by dilating pixels segmented as 'in situ tumor' using a disk-shaped structuring element with a radius of 6 units to create a binary mask representing the dilated region. The original 'in situ tumor' pixels are subtracted from this mask to define the border of the 'in situ tumor.' Another binary mask is created for all pixels segmented as 'tumor-associated stroma.' The overlap between the 'in situ tumor' border mask and the 'tumor-associated stroma' mask is determined by counting the number of overlapping pixels. The percentage of the 'in situ tumor' border occupied by 'tumor-associated stroma' is calculated by dividing the number of overlapping pixels by the total number of pixels in the 'in situ tumor' border.

The variance of healthy gland size feature represents the weighted coefficient of variation (CV) of the areas of the 'healthy epithelium' nests, within the whole tissue. This feature may be computed by first creating a binary mask is created where pixels segmented as 'healthy epithelium' are set to True and all other pixels are set to False. The binary mask is then labeled to identify connected regions (nests), where each labeled region corresponds to a continuous region of 'healthy epithelium' pixels. The area of each nest is determined by counting the number of pixels within it. Finally, the weighted coefficient of variation is calculated for these areas, with the weight for each nest being the nest area divided by the total area of all nests.

The inflammatory stroma area feature represents the total area of 'inflamed stroma' within the viable tumor. This feature may be computed by counting the number of pixels classified as 'inflamed stroma' that are located within the viable tumor.

The average nuclei size of stromal cells feature represents the average area of all tumor-associated stroma nuclei within the viable tumor. This feature may be computed by filtering nuclei to include only those located within the viable tumor and whose center coordinates align with pixels segmented as 'tumor-associated stroma.' The area of each nucleus is determined as the area enclosed by the contour provided by the instance segmentation algorithm. These areas are then averaged to compute the feature.

The mitotic hotspot count feature quantifies the number of circles (radius 564 micrometers) within the viable tumor area that contain 9 or more mitoses. This feature may be computed by drawing a circle with a radius of 564 micrometers around each pixel of the viable tumor. The number of mitoses within each circle is then counted. The feature value is calculated as the total count of these circles (referred to as "hotspots") that contain 9 or more mitoses.

The mitotic density feature represents the number of mitoses within the invasive tumor in the viable tumor, divided by the area of the invasive tumor in the viable tumor. This feature may be computed by counting those whose center coordinates overlap with pixels classified as 'invasive tumor.' This count is then divided by the total number of pixels segmented as 'invasive tumor' within the viable tumor.

Below section "Features computation" describes exemplary manners to compute the above features. Annex A provides a list of alternative manners to compute the above features. The

Applicant's test have shown that using these alternatives yields similar results to those obtained with the computations described above.

Once computed, all of these computed features may be stacked in the patient feature vector. The clinical features are derived from the patient clinical data, for example by directly stacking them in the patient feature vector. Alternatively, features are derived from the clinical features before stacking them in the patient feature vector.

As explained previously, the image annotation module 1210 and the feature vector calculation module 1220 work in the above described example hand in hand to constitute a feature extractor.

Once the patient feature vector is computed in operation 310, the risk calculation module 1230 may compute value quantifying a risk of relapse of breast cancer for a patient. Depending on the number of whole histological slide images, the risk calculation module 1230 may proceed in various manner. In an embodiment, the risk calculation module 1230 executes a machine learning module which is arranged to receive the patient feature vector and to return a numerical or binary value which quantifies the risk of relapse. The former case (binary value) is particularly useful where the patient data used as input contains a single whole histological slide image. The latter case (numerical value) is particularly useful where the patient data used as input contains several whole histological slide images. In an embodiment, the machine learning module is a L1-Cox model. In an alternative embodiment, the machine learning module can be an XGBoost (or another type of gradient boosted tree), a random forest, an SVM (Support Vector Machine), L2-Cox, or any type of regression model with a survival loss, as well as all classification models for predicting an event at a given timestep (e.g., at 5 years) including logistic regression. The man skilled in the art will note that all of these models can also be formulated as functions and such functions corresponding to these models will be understood as corresponding to the models.

Figure 4 shows an example of a function which details the operations of figure 3 for calculating the value which quantifies the risk of relapse.

In an operation 400, the patient data input PatDat is received as a set of whole histological slide images WHSI[] and a set of patient clinical data CD[].

A loop is then started for computing a list of risk scores for each whole histological slide image in WHSI[]. This is done by popping the list WHSI[] in an operation 410 to obtain an image Im. The image Im is then provided as input to the first annotation model in an operation 420, to the second annotation model in an operation 430, and to the third annotation model in an operation 440. Operations 420, 430 and 440 may be sequential, parallel, or a mix (two operations in parallel and one sequential before or after). The result is the annotation of image Im as described above. Thereafter, the feature calculation module 1220 computes the patient feature vector PatFv in an operation 450 using the annotated image Im and the clinical data CD[].

Once the patient feature vector PatFv is computed, a corresponding risk score can be determined by calling the risk calculation module of operation 330, as described above. This score is stored in a table RS[] in an operation 460 and the loop is repeated with operation 410. Once all whole histological slide images have been processed, and all scores RS[Im] corresponding to each of said image with the patient clinical data have been determined, the value which quantifies the risk of relapse RV can be determined in an operation 470. The corresponding function Rsk(V) can proceed in many manners: average of the RS[Im] values, weighed average, median, computation with selective dropouts, use of a restricted number of values (the minimas or the maximas), or a mix of all these methods. The goal is to use all the values generated by the different whole histological slide images to determine a patient risk value RV. Finally, this risk value can be compared to a threshold to determine whether the patient is at risk of relapse or not. In some embodiments, the values RS[Im] may be binary values.

It will appear readily that the above device can be used both for training and inference purposes in that it allows to determine the patient feature vectors which can be used to train the risk classifier with annotated data and to predict risk for new data.

### Materials and methods

The Applicant has tested the method and device described above, and has proven that it is more efficient than the state of the art relapse prediction methods.

A dataset of 1245 patients with breast cancer was collected. Each patient had 1 whole slide image (WSI) of their breast cancer, follow-up data including metastatic relapse, local relapse, regional relapse, death, and clinical data including number of positive lymph nodes, histological grade, tumor size, and percent of tumor cells positive to progesterone. Some patients had missing clinical values, which were imputed with 0.

Automatic annotations were extracted for all WSI for all three models. Then, the ten features were extracted. Each patient was therefore represented by a vector of fourteen features including the four clinical features and the ten histological features, as well as with their follow-up data.

A Cox proportional hazards model was instantiated. This model was trained five times in a fivefold cross-validation: for each fold, the Cox model was trained from scratch from the data of the patients in the training set ; once training was done, predictions were made for all patients of the fold testing set. Once all cross-validation folds training were done, all test predictions were gathered, and statistical analysis was performed. This statistical analysis included the measurement of the system performance, including computation of the concordance index, the continuous hazard ratio with 95% confidence interval, and the area under the ROC curve (AUC) at the time points of 5 years, 8 years and 10 years. Additionally, a hazard ratio was computed between binary predictions of the model using a generic cutoff at 20%: predictions below the 20^{th} percentile of predicted risks were assigned predicted label 1, while those higher or equal were assigned predicted label 0. This training and statistical analysis was done for multiple endpoints including metastatic relapse prediction, all relapse (metastatic, regional, local) prediction, metastatic relapse or death prediction, all relapse or death prediction, and death prediction. When reporting hazard ratios, the random performance is 1, and hazard ratios below 1 are better than random performance, while those above 1 are worse than random performance.

For metastatic relapse prediction, the system obtained a concordance index of 0.6983, a continuous hazard ratio of 0.64 (95% CI: 0.60-0.68, p<0.001), an AUC at 5 years of 0.744, an AUC at 8 years of 0.713, an AUC at 10 years of 0.703, and a binary hazard ratio of 0.37 (95% CI: 0.60-0.68; p<0.001).

For metastatic relapse or death prediction, the system obtained a concordance index of 0.6748, a continuous hazard ratio of 0.80 (95% CI: 0.75-0.86, p<0.001), an AUC at 5 years of 0.731, an AUC at 8 years of 0.692, an AUC at 10 years of 0.686, and a binary hazard ratio of 0.27 (95% CI: 0.18-0.40; p<0.001).

For all relapse (metastatic, regional, local) prediction, the system obtained a concordance index of 0.6331, a continuous hazard ratio of 0.64 (95% CI: 0.58-0.70, p<0.001), an AUC at 5 years of 0.654, an AUC at 8 years of 0.651, an AUC at 10 years of 0.648, and a binary hazard ratio of 0.39 (95% CI: 0.07-0.57; p<0.001).

For all relapse or death prediction, the system obtained a concordance index of 0.6418, a continuous hazard ratio of 0.72 (95% CI: 0.68-0.77, p<0.001), an AUC at 5 years of 0.676, an AUC at 8 years of 0.655, an AUC at 10 years of 0.664, and a binary hazard ratio of 0.30 (95% CI: 0.20-0.43; p<0.001).

For death prediction, the system obtained a concordance index of 0.6691, a continuous hazard ratio of 0.75 (95% CI: 0.71-0.80, p<0.001), an AUC at 5 years of 0.715, an AUC at 8 years of 0.688, an AUC at 10 years of 0.684, and a binary hazard ratio of 0.30 (95% CI: 0.19-0.46; p<0.001).

These findings were consistent with other machine learning models. For instance, the same protocol was applied with a random forest model. For metastatic relapse prediction, the system obtained a concordance index of 0.6929, a continuous hazard ratio of 0.50 (95% CI: 0.44-0.57, p<0.001), an AUC at 5 years of 0.737, an AUC at 8 years of 0.703, an AUC at 10 years of 0.702, and a binary hazard ratio of 0.25 (95% CI: 0.13-0.47; p<0.001).

Further experiments were conducted when multiple slides were available per patient. A Cox model was trained on the total dataset described above. A validation dataset of 251 patients was collected, where at least 2 slides were available for each patient. This dataset comprises a total of 515 slides for an average of 2.05 slides per patient. When the patient-level prediction was obtained with averaging predictions (i.e. slide predictions were averaged for each patient), the performance for metastatic relapse prediction was a concordance index of 0.7582, a continuous hazard ratio of 0.65 (95% CI: 0.58-0.72, p<0.001), an AUC at 5 years of 0.797, an AUC at 8 years of 0.753, an AUC at 10 years of 0.746, and a binary hazard ratio of 0.10 (95% CI: 0.01-0.0.73; p<0.001). When the patient-level prediction was obtained with taking the maximum prediction (i.e. the maximum slide prediction were taken for each patient), the performance for metastatic relapse prediction was a concordance index of 0.7466, a continuous hazard ratio of 0.17 (95% CI: 0.09-0.31, p<0.001), an AUC at 5 years of 0.782, an AUC at 8 years of 0.743, an AUC at 10 years of 0.736, and a binary hazard ratio of 0.15 (95% CI: 0.02-1.07; p=0.07).

The embodiments described herein were found to perform better than the traditional alternatives. Indeed, an experiment was conducted on the same development cohort as above, with a multiple instance learning-based approach. Instead of extracting features from slides as previously described, the following process was used for each slide. 100 random patches of size 224 pixels at 20x were extracted in the tissue region of the slide. Deep features were extracted using a ResNet50 convolutional neural network pretrained on ImageNet, resulting in 2048 deep features per patch. Average multiple instance learning was employed, meaning that the 100 patches were averaged channelwise, i.e. by averaging each feature dimension across all patches. This resulted in a vector of size 2048 for each slide. The same four clinicopathological variables were then concatenated for each patient, resulting in a final vector of size 2052. Then, a Cox model was trained in a cross-validation fashion, as was done for the embodiments described herein. For metastatic relapse prediction, the system obtained a concordance index of 0.5539 (vs 0.6983 for the proposed), a continuous hazard ratio of 0.93 (95% CI: 0.86-1.00, p=0.0449) (vs 0.64 (95% CI: 0.60-0.68, p<0.001) for the proposed), an AUC at 5 years of 0.583 (vs 0.744 for the proposed), an AUC at 8 years of 0.567 ((vs 0.713 for the proposed), an AUC at 10 years of 0.544 (vs 0.703 for the proposed), and a subrandom binary hazard ratio of 1.46 (95% CI: 0.76-2.82; p=0.241) (vs 0.37 (95% CI: 0.60-0.68; p<0.001) for the proposed).

Once a given patient has been determined as not risking a relapse, this information can be taken into consideration in their treatment, for example by using adapted patient care comprising hormone therapy and/or chemotherapy and/or cyclin-dependent kinase 4/6 and/or next-generation selective estrogen receptor degrader.

### ANNEX A

### Tumor area

The 'tumor area' can be implemented as:
● The ratio of the area of the invasive tumor to the area of the whole tissue. One possible way to implement it is: the invasive tumor area is computed as the total number of pixels segmented as 'invasive tumor' in the whole tissue. This area is then divided by the total number of pixels within the whole tissue.
● The area of the invasive tumor in the whole tissue. One possible way to implement it is: the invasive tumor area is computed as the total number of pixels segmented as 'invasive tumor' in the whole tissue.
● The average distance between all centroids of continuous regions ('nests') of 'invasive tumor' within the viable tumor. One possible way to implement it is: first, a binary mask is created where pixels segmented as 'invasive tumor' are set to True if they are within the viable tumor, and all other pixels are set to False. The binary mask is then labeled to identify connected regions (nests), where each labeled region corresponds to a continuous region of 'invasive tumor' pixels. All distances between nest centroids are computed and the feature is computed as the average of all these distances.
● The average minimal distance from 'invasive tumor' nuclei to 'tumor-associated stroma' nuclei within the viable tumor. One possible way to implement it is: for each nuclei segmented as 'invasive tumor', we compute its distance to each nuclei segmented as 'tumor-associated stroma' and we retain the minimal distance. The final feature is computed as the average of all these minimal distances.
● The number of 'invasive tumor' nuclei within the viable tumor. This is computed as the number of nuclei in the viable tumor, segmented as 'invasive tumor.'
● The number of 'tumor-associated stroma' nuclei within the viable tumor. This is computed as the number of nuclei in the viable tumor, segmented as 'tumor-associated stroma'.
● The area of the largest continuous region ('nest') of invasive tumor within the viable tumor. One possible way to implement it is: first, a binary mask is created where pixels segmented as 'invasive tumor' in the viable tumor are set to True and all other pixels are set to False. The binary mask is then labeled to identify connected regions (nests), where each labeled region corresponds to a continuous region of 'invasive tumor' pixels. Only the region with the highest area is kept and all other regions are set to False. Finally, the area of the largest region is computed as the total count of its pixels.
● The convex area of the largest continuous region ('nest') of invasive tumor within the viable tumor. One possible way to implement it is: first, a binary mask is created where pixels segmented as 'invasive tumor' in the viable tumor are set to True and all other pixels are set to False. The binary mask is then labeled to identify connected regions (nests), where each labeled region corresponds to a continuous region of 'invasive tumor' pixels. Only the region with the highest area is kept and all other regions are set to False. Finally, the convex area of the largest region is computed.
● The maximum Feret diameter of the largest continuous region ('nest') of invasive tumor within the viable tumor. One possible way to implement it is: first, a binary mask is created where pixels segmented as 'invasive tumor' in the viable tumor are set to True and all other pixels are set to False. The binary mask is then labeled to identify connected regions (nests), where each labeled region corresponds to a continuous region of 'invasive tumor' pixels. Only the region with the highest area is kept and all other regions are set to False. Finally, the maximum Feret diameter of the largest region is computed.
● The perimeter of the largest continuous region ('nest') of invasive tumor within the viable tumor. One possible way to implement it is: first, a binary mask is created where pixels segmented as 'invasive tumor' in the viable tumor are set to True and all other pixels are set to False. The binary mask is then labeled to identify connected regions (nests), where each labeled region corresponds to a continuous region of 'invasive tumor' pixels. Only the region with the highest area is kept and all other regions are set to False. Finally, the perimeter of the largest region is computed.
● The number of continuous regions ('nests') of 'tumor-associated stroma' within the viable tumor. One possible way to implement it is: first, a binary mask is created where pixels segmented as 'tumor-associated stroma' within the viable tumor are set to True and all other pixels are set to False. The binary mask is then labeled to identify connected regions (nests), where each labeled region corresponds to a continuous region of 'tumor-associated stroma' pixels. The total number of nests is determined by counting these labeled regions.
● The area of the viable tumor. This feature is computed as the number of pixels within the viable tumor.
● The maximum Feret diameter of the viable tumor. This feature is determined by measuring the maximum Feret diameter of the viable tumor.
● The perimeter of the viable tumor. This feature is determined by measuring the perimeter of the viable tumor.
● The ratio of the perimeter over the area of the viable tumor. This feature is determined by measuring the perimeter of the viable tumor divided by the area of the viable tumor.
● The area of the invasive tumor in the viable tumor. The invasive tumor area is computed as the total number of pixels segmented as 'invasive tumor' within the viable tumor.
● The total perimeter of 'invasive tumor' pixels within the viable tumor. The invasive tumor perimeter is computed as the sum of the perimeters of all 'invasive tumor' nests in the viable tumor.
● The area of the tumor-associated stroma in the viable tumor. The tumor-associated stroma area is computed as the total number of pixels segmented as 'tumor-associated stroma' within the viable tumor.
● The total perimeter of 'tumor-associated stroma' pixels within the viable tumor. The tumor-associated stroma perimeter is computed as the sum of the perimeters of all tumor-associated stroma nests within the viable tumor.
● The number of continuous regions ('nests') of 'tumor-associated stroma' divided by the area of the whole tissue. One possible way to implement it is: first, a binary mask is created where pixels segmented as 'tumor-associated stroma' in the whole tissue are set to True and all other pixels are set to False. The binary mask is then labeled to identify connected regions (nests), where each labeled region corresponds to a continuous region of 'tumor-associated stroma' pixels. The number of nests is determined by counting the labeled regions and is then divided by the area (sum of pixels) of the whole tissue to compute the feature.

### Tumor density at the invasive front

The 'tumor density at the invasive front' can be implemented as:
● The median of the distribution of distances from invasive tumor pixels to the border of the viable tumor, normalized by the median of the distances of all viable tumor pixels to the same border. One possible way to implement it is: the pixels forming the border of the viable tumor are first extracted. The distances of all invasive tumor pixels within the viable tumor to the border of the viable tumor are then computed, and the median of these distances is retrieved. Additionally, the distances of all pixels within the viable tumor to the border are computed, and the median of this distribution is extracted for normalization. The final feature is determined by dividing the median of the invasive tumor distance distribution by the median of the viable tumor distance distribution.
● The 75th percentile of the distribution of distances from invasive tumor pixels to the border of the viable tumor, normalized by the 75th percentile of distances of all viable tumor pixels to the same border. One possible way to implement it is: the pixels forming the border of the viable tumor are first extracted. The distances of all invasive tumor pixels to the border of the viable tumor are then computed, and the 75th percentile of these distances is retrieved. Additionally, the distances of all pixels within the viable tumor to the border are computed, and the 75th percentile of this distribution is extracted for normalization. The final feature is determined by dividing the 75th percentile of the invasive tumor distance distribution by the 75th percentile of the viable tumor distance distribution.

● The median of the distribution of distances between invasive tumor pixels, normalized by the median of distances between all viable tumor pixels. One possible way to implement it is: the distances between all 'invasive tumor' pixels within the viable tumor are computed and the median of this distribution is retrieved. Additionally, the distances between all pixels within the viable tumor are computed, and the median of this distribution is extracted for normalization. The final feature is determined by dividing the median of the invasive tumor distance distribution by the median of the viable tumor distance distribution.
● The 25th percentile of the distribution of distances between invasive tumor pixels, normalized by the 25th percentile of the distribution of distances between all viable tumor pixels. One possible way to implement it is: the distances between all 'invasive tumor' pixels within the viable tumor are computed and the 25th percentile of this distance distribution is retrieved. Additionally, the distances between all pixels within the viable tumor are computed, and the 25th percentile of this distribution is extracted for normalization. The final feature is determined by dividing the 25th percentile of the invasive tumor distance distribution by the 25th percentile of the viable tumor distance distribution.

### Invasive tumor nest density

The 'invasive tumor nest density' can be implemented as:
- The number of continuous regions ('nests') of 'invasive tumor' within the viable tumor. One possible way to implement it is: first, a binary mask is created where pixels segmented as 'invasive tumor' within the viable tumor are set to True and all other pixels are set to False. The binary mask is then labeled to identify connected regions (nests), where each labeled region corresponds to a continuous region of 'invasive tumor' pixels. The total number of nests is determined by counting these labeled regions.
- The number of continuous regions ('nests') of 'invasive tumor' within the whole tissue. One possible way to implement it is: first, a binary mask is created where pixels segmented as 'invasive tumor' in the whole tissue are set to True and all other pixels are set to False. The binary mask is then labeled to identify connected regions (nests), where each labeled region corresponds to a continuous region of 'invasive tumor' pixels. The total number of nests is determined by counting these labeled regions.

### In situ tumor nest density

The 'in situ tumor nest density' can be implemented as:
● The proportion of in situ tumor within the viable tumor. One possible way to implement it is: first, the area of in situ tumor is computed as the sum of all pixels segmented as 'in situ tumor' within the viable tumor. This area is then divided by the total viable tumor area to obtain the proportion of in situ tumor.
● The number of continuous regions ('nests') of in situ tumor within the viable tumor. One possible way to implement it is: first, a binary mask is created where pixels segmented as 'in situ tumor' within the viable tumor are set to True, while all other pixels are set to False. The binary mask is then labeled to identify connected regions (nests), where each labeled region corresponds to a continuous region of in situ tumor pixels. The total number of nests is determined by counting these labeled regions.
● The ratio between the area of 'adipocyte tissue' and the area of 'in situ tumor' within the viable tumor. One possible way to implement it is: the area of adipocyte is computed as the sum of all pixels segmented as 'adipocyte tissue within the viable tumor. Similarly, the area of in situ tumor is computed as the sum of all pixels segmented as 'in situ tumor' within the viable tumor. The ratio is then obtained by dividing the adipocyte area by the in situ tumor area.
● The ratio between the area of 'invasive tumor' and the area of 'in situ tumor' within the viable tumor One possible way to implement it is: the area of invasive tumor is computed as the sum of all pixels segmented as 'invasive tumor' within the viable tumor. Similarly, the area of in situ tumor is computed as the sum of all pixels segmented as 'in situ tumor' within the viable tumor. The ratio is then obtained by dividing the invasive tumor area by the in situ tumor area.
● The ratio between the area of 'tumor-associated stroma' and the area of 'in situ tumor' within the viable tumor. One possible way to implement it is: first, the area of tumor-associated stroma is computed as the sum of all pixels segmented as 'tumor-associated stroma' within the viable tumor. Similarly, the area of in situ tumor is computed as the sum of all pixels segmented as 'in situ tumor' within the viable tumor. The ratio is then obtained by dividing the tumor-associated stroma area by the in situ tumor area.

### Healthy gland size variance

The 'healthy gland size variance' can be implemented as:
● The weighted coefficient of variation of the convex areas of continuous regions ('nests') of healthy glands in the whole tissue. One possible way to implement it is: first, a binary mask is created where pixels segmented as 'healthy epithelium' in the whole tissue are set to True, while all other pixels are set to False. The binary mask is then labeled to identify connected regions (nests), where each labeled region corresponds to a continuous region of 'healthy epithelium' pixels. The convex areas of each nest are computed. The weighted coefficient of variation of the areas is then calculated as the weighted standard deviation of the areas divided by the weighted mean of the areas.
● The weighted coefficient of variation of the compactness of continuous regions ('nests') of healthy glands in the whole tissue. One possible way to implement it is: first, a binary mask is created where pixels segmented as 'healthy epithelium' in the whole tissue are set to True, while all other pixels are set to False. The binary mask is then labeled to identify connected regions (nests), where each labeled region corresponds to a continuous region of 'healthy epithelium' pixels. The compactness of each nest is computed (perimeter*perimeter/area). The weighted coefficient of variation of the compactness values is then calculated as the weighted standard deviation of compactness divided by the weighted mean of compactness.
● The weighted coefficient of variation of the maximum Feret diameter of continuous regions ('nests') of healthy glands in the whole tissue. One possible way to implement it is: first, a binary mask is created where pixels segmented as 'healthy epithelium' in the whole tissue are set to True, while all other pixels are set to False. The binary mask is then labeled to identify connected regions (nests), where each labeled region corresponds to a continuous region of 'healthy epithelium' pixels. The maximum Feret diameter is computed for each nest. The weighted coefficient of variation of the maximum Feret diameters is then calculated as the weighted standard deviation of the maximum Feret diameters divided by the weighted mean of the maximum Feret diameters.
● The weighted coefficient of variation of the perimeters of continuous regions ('nests') of healthy glands in the whole tissue. One possible way to implement it is: first, a binary mask is created where pixels segmented as 'healthy epithelium' in the whole tissue are set to True, while all other pixels are set to False. The binary mask is then labeled to identify connected regions (nests), where each labeled region corresponds to a continuous region of 'healthy epithelium' pixels. The perimeter of each nest is computed. The weighted coefficient of variation of the perimeters is then calculated as the weighted standard deviation of the perimeters divided by the weighted mean of the perimeters.
● The area of the largest continuous region ('nest') of healthy glands within the whole tissue. One possible way to implement it is: first, a binary mask is created where pixels segmented as 'healthy epithelium' in the whole tissue are set to True and all other pixels are set to False. The binary mask is then labeled to identify connected regions (nests), where each labeled region corresponds to a continuous region of 'healthy epithelium' pixels. Only the region with the highest area is kept and all other regions are set to False. Finally, the area of the largest region is computed as the total count of its pixels.
● The convex area of the largest continuous region ('nest') of healthy glands within the whole tissue. One possible way to implement it is: first, a binary mask is created where pixels segmented as 'healthy epithelium' in the whole tissue are set to True and all other pixels are set to False. The binary mask is then labeled to identify connected regions (nests), where each labeled region corresponds to a continuous region of 'healthy epithelium' pixels. Only the region with the highest area is kept and all other regions are set to False. Finally, the convex area of the largest region is computed.
● The circularity of the largest continuous region ('nest') of healthy glands within the whole tissue. One possible way to implement it is: first, a binary mask is created where pixels segmented as 'healthy epithelium' in the whole tissue are set to True and all other pixels are set to False. The binary mask is then labeled to identify connected regions (nests), where each labeled region corresponds to a continuous region of 'healthy epithelium' pixels. Only the region with the highest area is kept and all other regions are set to False. Finally, the circularity of the largest region is computed using the formula: 4π×area / perimeter*perimeter.
● The compactness of the largest continuous region ('nest') of healthy glands within the whole tissue. One possible way to implement it is: first, a binary mask is created where pixels segmented as 'healthy epithelium' in the whole tissue are set to True and all other pixels are set to False. The binary mask is then labeled to identify connected regions (nests), where each labeled region corresponds to a continuous region of 'healthy epithelium' pixels. Only the region with the highest area is kept and all other regions are set to False. Finally, the compactness of the largest region is computed using the formula: perimeter*perimeter / area
● The maximum Feret diameter of the largest continuous region ('nest') of healthy glands within the whole tissue. One possible way to implement it is: first, a binary mask is created where pixels segmented as 'healthy epithelium' in the whole tissue are set to True and all other pixels are set to False. The binary mask is then labeled to identify connected regions (nests), where each labeled region corresponds to a continuous region of 'healthy epithelium' pixels. Only the region with the highest area is kept and all other regions are set to False. Finally, the maximum Feret diameter of the largest region is computed.
● The perimeter of the largest continuous region ('nest') of healthy glands within the whole tissue. One possible way to implement it is: first, a binary mask is created where pixels segmented as 'healthy epithelium' in the whole tissue are set to True and all other pixels are set to False. The binary mask is then labeled to identify connected regions (nests), where each labeled region corresponds to a continuous region of 'healthy epithelium' pixels. Only the region with the highest area is kept and all other regions are set to False. Finally, the perimeter of the largest region is computed.

### Inflammatory stroma area

The 'inflammatory stroma area' can be implemented as:
● The number of 'inflamed stroma' nuclei. This feature is computed as the number of nuclei in the viable tumor, segmented as inflamed stroma'.
● The total perimeter of 'inflamed stroma' within the viable tumor. The feature is computed as the sum of the perimeters of all inflamed stroma nests within the viable tumor.
● The total area of 'inflamed stroma' in the whole tissue. The feature is computed by counting the number of pixels classified as 'inflamed stroma' that are located in the whole tissue.
● The total perimeter of 'inflamed stroma' in the whole tissue. The feature is computed as the sum of the perimeters of all inflamed stroma nests in the whole tissue.
● The weighted mean of the convex areas of continuous regions ('nests') of inflamed stroma within the viable tumor.One possible way to implement it is: first, a binary mask is created where pixels segmented as 'inflamed stroma' in the viable tumor are set to True, while all other pixels are set to False. The binary mask is then labeled to identify connected regions (nests), where each labeled region corresponds to a continuous region of 'inflamed stroma' pixels. The convex area of each nest is computed and weighted by the proportion of its area relative to the total area of all inflamed stroma nests. The weighted mean of the convex areas is then calculated as the sum of all convex areas, each multiplied by its respective weight.
● The weighted mean of the areas of continuous regions ('nests') of inflamed stroma within the viable tumor. One possible way to implement it is: first, a binary mask is created where pixels segmented as 'inflamed stroma' in the viable tumor are set to True, while all other pixels are set to False. The binary mask is then labeled to identify connected regions (nests), where each labeled region corresponds to a continuous region of 'inflamed stroma' pixels. The area of each nest is computed and weighted by the proportion of its area relative to the total area of all 'inflamed stroma' nests. The weighted mean of the areas is then calculated as the sum of all areas, each multiplied by its respective weight.
● The weighted mean of the compactness of continuous regions ('nests') of inflamed stroma within the viable tumor. One possible way to implement it is: first, a binary mask is created where pixels segmented as 'inflamed stroma' in the viable tumor are set to True, while all other pixels are set to False. The binary mask is then labeled to identify connected regions (nests), where each labeled region corresponds to a continuous region of 'inflamed stroma' pixels. The compactness (perimeter*perimeter / area) of each nest is computed and weighted by the proportion of its area relative to the total area of all 'inflamed stroma' nests. The weighted mean of the compactnesses is then calculated as the sum of all compactnesses, each multiplied by its respective weight.
● The weighted mean of the maximum Feret diameter of continuous regions ('nests') of inflamed stroma within the viable tumor. One possible way to implement it is: first, a binary mask is created where pixels segmented as 'inflamed stroma' in the viable tumor are set to True, while all other pixels are set to False. The binary mask is then labeled to identify connected regions (nests), where each labeled region corresponds to a continuous region of 'inflamed stroma' pixels. The maximum Feret diameter of each nest is computed and weighted by the proportion of its area relative to the total area of all 'inflamed stroma' nests. The weighted mean of the maximum Feret diameter is then calculated as the sum of all maximum Feret diameters, each multiplied by its respective weight.
● The weighted mean of the perimeter of continuous regions ('nests') of inflamed stroma within the viable tumor. One possible way to implement it is: first, a binary mask is created where pixels segmented as 'inflamed stroma' in the viable tumor are set to True, while all other pixels are set to False. The binary mask is then labeled to identify connected regions (nests), where each labeled region corresponds to a continuous region of 'inflamed stroma' pixels. The perimeter of each nest is computed and weighted by the proportion of its area relative to the total area of all 'inflamed stroma' nests. The weighted mean of the perimeters is then calculated as the sum of all perimeters, each multiplied by its respective weight.
● The proportion of inflamed stroma within the viable tumor. One possible way to implement it is: first, the area of inflamed stroma is computed as the sum of all pixels segmented as inflamed stroma within the viable tumor. This area is then divided by the total viable tumor area to obtain the proportion of inflamed stroma.
● The area of the largest continuous region ('nest') of inflamed stroma within the viable tumor. One possible way to implement it is: first, a binary mask is created where pixels segmented as 'inflamed stroma' in the viable tumor are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the area of the largest region is computed as the total count of its pixels.
● The convex area of the largest continuous region ('nest') of inflamed stroma within the viable tumor. One possible way to implement it is: first, a binary mask is created where pixels segmented as 'inflamed stroma' in the viable tumor are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the convex area of the largest region is computed.
● The circularity of the largest continuous region ('nest') of inflamed stroma within the viable tumor. One possible way to implement it is: first, a binary mask is created where pixels segmented as 'inflamed stroma' in the viable tumor are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the circularity of the largest region is computed using the formula: 4π×area / perimeter*perimeter.
● The compactness of the largest continuous region ('nest') of inflamed stroma within the viable tumor. One possible way to implement it is: first, a binary mask is created where pixels segmented as 'inflamed stroma' in the viable tumor are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the compactness of the largest region is computed using the formula: perimeter*perimeter / area.
● The maximum Feret diameter of the largest continuous region ('nest') of inflamed stroma within the viable tumor. One possible way to implement it is: first, a binary mask is created where pixels segmented as 'inflamed stroma' in the viable tumor are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the maximum Feret diameter of the largest region is computed.
● The perimeter of the largest continuous region ('nest') of inflamed stroma within the viable tumor. One possible way to implement it is: first, a binary mask is created where pixels segmented as 'inflamed stroma' in the viable tumor are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the perimeter of the largest region is computed.
● The number of continuous regions ('nests') of inflamed stroma within the viable tumor.One possible way to implement it is: first, a binary mask is created where pixels segmented as inflamed stroma within the viable tumor are set to True, while all other pixels are set to False. The binary mask is then labeled to identify connected regions (nests), where each labeled region corresponds to a continuous region of inflamed stroma pixels. The total number of nests is determined by counting these labeled regions.
● The percentage of the border of 'invasive tumor' that is occupied by 'inflamed stroma'. One possible way to implement it is: pixels segmented as 'invasive tumor' in the viable tumor are dilated using a disk-shaped structuring element with a radius of 15 units to create a binary mask representing the dilated region. The original 'invasive tumor' pixels are subtracted from this mask to define the border of the 'invasive tumor.' Another binary mask is created for all pixels segmented as 'inflamed stroma.' We then take the intersection of the 'invasive tumor' border mask and the 'inflamed stroma' mask. The percentage of the 'invasive tumor' border occupied by 'inflamed stroma' is calculated by dividing the number of 'inflamed stroma' pixels in the overlap divided by the total number of pixels in the 'invasive tumor' border.
● The percentage of the border of 'invasive tumor' that is occupied by 'inflamed stroma'. One possible way to implement it is: pixels segmented as 'invasive tumor' in the viable tumor are dilated using a disk-shaped structuring element with a radius of 6 units to create a binary mask representing the dilated region. The original 'invasive tumor' pixels are subtracted from this mask to define the border of the 'invasive tumor.' Another binary mask is created for all pixels segmented as 'inflamed stroma.' We then take the intersection of the 'invasive tumor' border mask and the 'inflamed stroma' mask. The percentage of the 'invasive tumor' border occupied by 'inflamed stroma' is calculated by dividing the number of 'inflamed stroma' pixels in the overlap divided by the total number of pixels in the 'invasive tumor' border.
● The weighted mean of the convex areas of continuous regions ('nests') of inflamed stroma in the whole tissue. One possible way to implement it is: first, a binary mask is created where pixels segmented as 'inflamed stroma' in the whole tissue are set to True, while all other pixels are set to False. The binary mask is then labeled to identify connected regions (nests), where each labeled region corresponds to a continuous region of 'inflamed stroma' pixels. The convex area of each nest is computed and weighted by the proportion of its area relative to the total area of all nests. The weighted mean of the convex areas is then calculated as the sum of all convex areas, each multiplied by its respective weight.
● The weighted mean of the perimeters of continuous regions ('nests') of inflamed stroma in the whole tissue. One possible way to implement it is: first, a binary mask is created where pixels segmented as 'inflamed stroma' in the whole tissue are set to True, while all other pixels are set to False. The binary mask is then labeled to identify connected regions (nests), where each labeled region corresponds to a continuous region of 'inflamed stroma' pixels. The perimeter of each nest is computed and weighted by the proportion of its area relative to the total area of all 'inflamed stroma' nests. The weighted mean of the perimeters is then calculated as the sum of all perimeters, each multiplied by its respective weight.
● The area of the largest continuous region ('nest') of inflamed stroma within the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the area of the largest region is computed as the total count of its pixels.
● The convex area of the largest continuous region ('nest') of inflamed stroma within the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the convex area of the largest region is computed.
● The circularity of the largest continuous region ('nest') of inflamed stroma within the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the circularity of the largest region is computed using the formula: 4π×area / perimeter*perimeter.
● The compactness of the largest continuous region ('nest') of inflamed stroma within the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the compactness of the largest region is computed using the formula: perimeter*perimeter / area.
● The maximum Feret diameter of the largest continuous region ('nest') of inflamed stroma within the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the maximum Feret diameter of the largest region is computed.
● The perimeter of the largest continuous region ('nest') of inflamed stroma within the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of 'inflamed stroma' pixels. Only the region with the highest area is kept and all other regions are set to False. Finally, the perimeter of the largest region is computed.
● The number of continuous regions ('nests') of inflamed stroma within the whole tissue. One possible way to implement it is: first, a binary mask is created where pixels segmented as inflamed stroma in the whole tissue are set to True, while all other pixels are set to False. The binary mask is then labeled to identify connected regions (nests), where each labeled region corresponds to a continuous region of inflamed stroma pixels. The total number of nests is determined by counting these labeled regions.
● The number of continuous regions ('nests') of inflamed stroma divided by the area of the whole tissue. One possible way to implement it is: first, a binary mask is created where pixels segmented as 'inflamed stroma' in the whole tissue are set to True and all other pixels are set to False. The binary mask is then labeled to identify connected regions (nests), where each labeled region corresponds to a continuous region of inflamed stroma pixels. The number of nests is determined by counting the unique labeled regions and is then divided by the area (sum of pixels) of the whole tissue to compute the feature.

### Nuclei size of stromal cells

The 'nuclei size of stromal cells' can be implemented as:
- The average perimeter of all tumor-associated stroma nuclei within the viable tumor. One possible way to implement it is: nuclei are filtered to include only those located within the viable tumor and whose center coordinates align with pixels segmented as 'tumor-associated stroma.' The perimeter of each nucleus is determined as the perimeter of the contour provided by the instance segmentation algorithm. These perimeters are then averaged to compute the feature.

### Mitotic hotspot count

The 'mitotic hotspot count' can be implemented as:
- The number of circles (radius 977 micrometers) within the viable tumor area that contain 10 or more mitoses. One possible way to implement it is: a circle with a radius of 977 micrometers is drawn around each pixel of the viable tumor. The number of mitoses within each circle is then counted. The feature value is calculated as the total count of these circles (referred to as "hotspots") that contain 10 or more mitoses.
- The number of circles (radius 564 micrometers) within the viable tumor area that contain 3 or more mitoses. One possible way to implement it is: a circle with a radius of 564 micrometers is drawn around each pixel of the viable tumor. The number of mitoses within each circle is then counted. The feature value is calculated as the total count of these circles (referred to as "hotspots") that contain 3 or more mitoses.

### Mitotic density

The 'mitotic density' can be implemented as:
● The maximum mitosis density among circles (radius 977 micrometers) within the viable tumor. One possible way to implement it is: a circle with a radius of 977 micrometers is drawn around each pixel of the viable tumor. The number of mitoses divided by the 'invasive tumor' area within the circle is then computed for each circle. The final feature value represents the maximum of these mitosis densities.
● The maximum mitosis density among circles (radius 564 micrometers) within the viable tumor. One possible way to implement it is: a circle with a radius of 564 micrometers is drawn around each pixel of the viable tumor. The number of mitoses divided by the 'invasive tumor' area within the circle is then computed for each circle. The final feature value represents the maximum of these mitosis densities.
● The average minimal distance between mitoses within the viable tumor. One possible way to implement it is: for each mitosis within the viable tumor, we compute its distance to each other mitosis and keep the minimal distance. The final feature is computed as the average of all these minimal distances.
● The total number of mitoses within the viable tumor. One possible way to implement it is: the total number of mitoses within the viable tumor is counted and recorded as the final feature.
● The number of mitoses per nucleus. One possible way to implement it is: the total number of mitosis within the viable tumor is counted and the total number of nuclei segmented as 'invasive tumor' within the viable tumor is counted. We then divided the total number of mitoses by the number of nuclei.

## Claims

1. Device for determining a value quantifying a risk of relapse of breast cancer for a patient comprising:
- a data storage (114) adapted to store patient data associating, for each given patient at a least one whole histological slide image and patient clinical data comprising at least a tumor size, a number of positive lymph nodes, a tumor grade, and progesterone receptor (PR) positivity value,
- a feature extractor (1210, 1220) arranged to receive a whole histological slide image and associated patient clinical data, to derive tumor architecture features comprising a tumor area, an invasive tumor nest density, a tumor density at the invasive front, and an in situ tumor nest density, tumor microenvironment features comprising a border composition of in situ tumor, a variance of healthy gland size, an inflammatory stroma area, and an average nuclei size of stromal cells, and mitosis features comprising a mitotic hotspot count and a mitotic density, and to return a patient feature vector comprising said tumor architecture features, said tumor microenvironment features, said mitosis features, and features derived from said associated patient clinical data,
- a risk classifier (1230) using a machine learning module which is arranged to receive a patient feature vector as an input and to return a value quantifying a risk of relapse of breast cancer for a patient, said machine learning module having been trained with a dataset of labelled patient feature vectors and being arranged to return a risk value,
said device being arranged to receive a set of patient data of a given patient, to provide at least some of said given patient's whole histological slide image and said given patient's clinical data as inputs to the feature extractor, to provide at least some of the resulting patient feature vectors to the risk classifier (1230), and to return a value quantifying a risk of relapse of breast cancer for a patient for said given patient based on the outputs of the risk classifier (1230).

2. Device according to claim 1, in which said feature extractor (1210, 1220) comprises :
- a first annotation model comprising a deep learning neural network arranged to receive a whole histological slide image as an input image and to return a class identifier for each pixel of the input image, said class identifier being chosen in a group comprising non-tissue, invasive tumor, in situ tumor, healthy epithelium, necrosis, inflamed stroma, tumor-associated stroma, adipocyte tissue and unclassified tissue,
- a second annotation model comprising a deep learning neural network arranged to receive a whole histological slide image as an input image and to return data identifying cell nuclei in the input image in the form of a nuclei identifier attached to each pixel of the input image and/or a set of nuclei bounding boxes, and
- a third annotation model comprising a third deep learning neural network arranged to receive a whole histological slide image as an input image and to return data identifying cell mitosis in the input image in the form of a nuclei identifier attached to each pixel of the input image and/or a set of mitosis bounding boxes, and
- a feature calculator arranged to derive said tumor architecture features, said tumor microenvironment features, said mitosis features from the outputs of said first annotation model, said second annotation model and said third annotation model provided with the whole histological slide image received by the feature extractor (1210, 1220).

3. Device according to claim 2, in which the first annotation model comprises an attention U-Net model with a MobileNet_v3 encoder.

4. Device according to claim 2 or 3, in which the second annotation model comprises a NuLite model.

5. Device according to one of claims 2 to 4, in which the third annotation model comprises a Fully Convolutional One-Stage (FCOS) Object Detection model with a ResNet-50 encoder.

6. Device according to one of the preceding claims, in which said patient clinical data further comprises one or more of a Ki-67 proliferation index, an estrogen receptor (ER) status, an estrogen receptor (ER) positivity value, a progesterone receptor (PR) status, a HER2 intensity (HER2-) value, a pathological T stage, pathological N stage , a menopausal status, a type of surgery, and a histological subtype.

7. Device according to one of the preceding claims, in which, when the patient data comprises more than one whole histological slide image, the device is arranged to derive a patient risk value from each risk value derived from the more than one whole histological slide images and to determine a value quantifying a risk of relapse of breast cancer for a patient by comparing said patient risk value to a threshold value, and, when the patient data comprises one whole histological slide image to determine a value quantifying a risk of relapse of breast cancer for a patient by comparing said risk value derived from said one whole histological slide image to a threshold value.

8. Device according to claim 7, in which, when the patient data comprises more than one whole histological slide image, the device is arranged to determine said patient risk value by performing one or more of the following operations : discarding certain risk values based on their corresponding patient feature vector, discarding certain risk values based on their value, averaging certain risk values, weighed averaging certain risk values.

9. Device according to one of the preceding claims, in which the machine learning module of the risk classifier (1230) comprises a Cox model or a gradient boosted tree type model or a Support Vector Machine or a random forest model.

10. Device for training a machine learning module for use in a device according to one of claims 1 to 9, comprising:
- a data storage for receiving a training dataset comprising patient feature vectors each labelled with a corresponding risk value,
- a machine learning module comprising a Cox model or a gradient boosted tree type model or a Support Vector Machine or a random forest model arranged to receive a patient feature vector and to return a risk value,
said device being arranged to train said machine learning module with said training dataset.

11. Method for determining a value quantifying a risk of relapse of breast cancer for a patient comprising:
a) receiving a set of patient data of a given patient, said patient data comprising at a least one whole histological slide image and patient clinical data comprising at least a tumor size, a number of positive lymph nodes, a tumor grade, and progesterone receptor (PR) positivity value,
b) for at least some of said at least one whole histological slide image, deriving tumor architecture features comprising a tumor area, an invasive tumor nest density, a tumor density at the invasive front, and an in situ tumor nest density, tumor microenvironment features comprising a border composition of in situ tumor, a variance of healthy gland size, an inflammatory stroma area, and an average nuclei size of stromal cells, and mitosis features comprising a mitotic hotspot count and a mitotic density, and returning each time a patient feature vector comprising said tumor architecture features, said tumor microenvironment features, said mitosis features, and features derived from said patient clinical data,
c) providing said patient feature vectors to a machine learning module having been trained with a dataset of labelled patient feature vectors and being arranged to return a risk value quantifying a risk of relapse of breast cancer for a patient, and returning a value quantifying a risk of relapse of breast cancer for a patient for said given patient based on the risk values determined from said patient feature vectors.

12. Method according to claim 11, in which operation b) comprises, for said at least some of said at least one whole histological slide image:
b1) determining a class identifier for each pixel of the input image, said class identifier being chosen in a group comprising non-tissue, invasive tumor, in situ tumor, healthy epithelium, necrosis, inflamed stroma, tumor-associated stroma, adipocyte tissue and unclassified tissue,
data identifying cell nuclei in the input image in the form of a nuclei identifier attached to each pixel of the input image and/or a set of nuclei bounding boxes, and data identifying cell mitosis in the input image in the form of a nuclei identifier attached to each pixel of the input image and/or a set of mitosis bounding boxes, and
b2) deriving said tumor architecture features, said tumor microenvironment features, said mitosis features from the output said at least some of said at least one whole histological slide image and the output of operation b1).

13. Method according to claim 11 or 12, in which operation c) comprises, when the patient data comprises more than one whole histological slide image, deriving a patient risk value from each risk value determined from said patient feature vectors and determining a value quantifying a risk of relapse of breast cancer for a patient by comparing said patient risk value to a threshold value, and, when the patient data comprises one whole histological slide image, determining a value quantifying a risk of relapse of breast cancer for a patient by comparing said risk value derived from said one whole histological slide image to a threshold value.

14. Method according to claim 13, in which operation c) comprises, when the patient data comprises more than one whole histological slide image, determining said patient risk value by performing one or more of the following operations : discarding certain risk values based on their corresponding patient feature vector, discarding certain risk values based on their value, averaging certain risk values, weighed averaging certain risk values.

15. A non-transitory program storage device comprising instructions which, when executed by a computer, cause the computer to carry out the method of claims 11 to 14.
